# EUROPEAN PATENT APPLICATION

(11) **EP 2 728 343 A1**
(43) Date of publication of application: **07.05.2014**
(21) Application number: 12191447.7
(22) Date of filing: 06.11.2012
(51) Int. Cl.: G01N 21/64, G01N 33/58, G01N 31/22, G01N 21/77

(54) **Optical probe for quantitatively determining an analyte**

(71) Applicant: Technische Universität Graz, 8010 Graz (AT)
(72) Inventor: Klimant, Ingo, 8200 Labuch (AT); Borisov, Sergey M., 8010 Graz (AT)
(74) Representative: Patentanwaltskanzlei Matschnig & Forsthuber OG

(57) **Abstract**

In an optical probe for quantitatively determining an analyte, the probe comprising a luminescent solid state material as an internal light source having an excitation spectrum (1) and an emission spectrum (2) and an indicator dye having different, analyte-dependent chemical forms and corresponding analyte-dependent absorption spectra (3,4), wherein the absorption spectrum of at least one form of the indicator dye overlaps with either the excitation spectrum or the emission spectrum of the luminescent solid state material, referred to as a probing spectrum, and the luminescent solid state material has a probing spectrum broader than the absorption spectrum of the indicator dye, by virtue of the indicator dye a first portion and a second portion of the probing spectrum of the luminescent solid state material are quenched to different extents, and wherein the decay time of the emission of the luminescent solid state material is longer than 1 µs.

## Description

The invention relates to an optical probe and a method for quantitatively determining an analyte.

Optical probes for various analytes such as oxygen, carbon dioxide, protons (for pH-measurements) and other ions are nowadays widely used in science and technology. Such probes, also referred to as optical chemosensors mostly rely on absorption indicator dyes or luminescent solid state materials which respond to the analyte of interest by changing their optical properties, such as absorption- or emission spectrum, luminescence decay time or luminescence anisotropy. The common problem of the absorption/reflectance sensors is that the light from the light source used to excite the luminescent solid state material is not only absorbed and reflected on the sensor surface but also on all the components of the optical equipment (e.g. connections between filters and fibres). This interference is difficult to quantify, which explains the popularity of the luminescence-based systems. Additionally, such luminescence-based probes or sensors are suitable for a broader range of applications (e.g. imaging). Luminescence intensity as such is a problematic parameter to measure since it is strongly influenced by a number of factors such as the intensity of the excitation light and its distance to the sensor, the sensitivity of the photodetector, scattering and coloration and turbidity in the specimen to be measured.

This disadvantage can be overcome by measuring luminescence decay time. Measurement of the luminescence decay time is very common in case of oxygen sensors which rely on long-lived phosphorescent solid state materials. However, the equipment becomes more bulky and expensive for measuring fluorescence decay time. Here, ratiometric measurement of the luminescence intensity is often preferable. To do so, typically, a luminescent (analyte-insensitive) solid state material is immobilised along with an indicator dye. Both, the indicator dye and the luminescent solid state material are excitable together, but possess substantially different emission spectra. The emission ratio of the indicator dye and the luminescent solid state material serves as an analytical parameter. Alternatively, a probe which possesses two different emission spectra in the absence and in the presence of the analyte can be used.

Optical probes or sensors based on the above referencing schemes possess several limitations: first, the interferences from background fluorescence of the probe is not eliminated; second, the presence of luminescence quenchers in the analysed media can severely affect the response of the sensors; third, the number of fluorescent indicators which fulfil the requirements of brightness, sensitivity, solubility in the sensor matrices, chemical- and photostability is rather limited. On the other hand, many robust absorption-based indicators are known. The use of such indicators can overcome the above drawbacks providing that a robust referencing scheme is found.

Several methods based on the use of absorption-based indicator dyes with luminescent read-out have been proposed. In the first one, a luminescent solid state material which possesses long-lived luminescence is dissolved in a polymer matrix along with an absorption-based indicator dye. Importantly, the emission of the luminescent solid state material overlaps with the absorption of one chemical form of the indicator dye (for example protonated or deprotonated form in case of a pH indicator) so that the luminescence intensity, and also the decay time decrease due to Förster Resonance Energy Transfer (FRET). Since the other form of the absorption-based indicator dye does not overlap with the emission spectrum of the luminescent solid state material, the FRET is absent in this case. The drawback of this method is severe cross-sensitivity to oxygen of the referenced dyes which possess long-lived luminescence. Ionic species (which in case of the pH and other ion sensors freely diffuse into the matrix polymer) can also quench the luminescence.

Another conversion scheme proposed is based on the so called "inner filter effect". In contrast to the previous scheme a luminescent solid state material can be immobilised into inert gas-impermeable material so that the above mentioned cross-sensitivities are avoided. The luminescence of the solid state material is reabsorbed by the indicator. Although this principle does allow discrimination of autofluorescence in time-resolved measurements, it is still prone to all the drawbacks of the intensity-based measurements. In fact, the absorption changes are converted to the changes of phosphorescence intensity measured at a single wavelength.

There is, thus, a need to provide an optical probe and a method for quantitatively determining an analyte that avoid the above described limitations and drawbacks.

To solve this object there is provided an optical probe for quantitatively determining an analyte, the probe comprising
a luminescent solid state material as an internal light source having an excitation spectrum and an emission spectrum and
an indicator dye having different, analyte-dependent chemical forms and corresponding analyte-dependent absorption spectra,
wherein the absorption spectrum of at least one form of the indicator dye overlaps with either the excitation spectrum or the emission spectrum of the luminescent solid state material, referred to as a probing spectrum, and the luminescent solid state material has a probing spectrum broader than the absorption spectrum of the indicator dye,
wherein
by virtue of the indicator dye, a first portion and a second portion of the probing spectrum of the luminescent solid state material are quenched to different extents, and wherein the decay time of the emission of the luminescent solid state material is longer than 1µs.

Such an optical probe allows for ratiometric measurement of an analyte by the analyte-dependent quenching of the probing spectrum of a luminescent solid state material at different wavelengths by the indicator dye. In the context of the invention, the term "quenching" is used for extinction of light due to inner filter effect between the luminescent solid state material and the indicator dye. Since the probing spectrum is overlapped only in part by an absorption spectrum of one analyte-dependent chemical form of the indicator dye, the indicator dye quenches the probing spectrum differently in different portions of the probing spectrum, the ratio of quenching is only dependent on the concentration of the analyte. The ratio of quenching is not affected by outer influences even when the absolute values measured are influenced by scattering, reflectance of the probe and the like. Thus the inventive optical probe allows for robust, standardised quantitative determination of an analyte's concentration without having to take into account external influences of the medium in which the measurement is carried out. The relatively long decay time of the luminescent solid state material allows for a time resolved measurement of the emission light independent from the excitation.

Ideally, the luminescent solid state material and the indicator dye would be selected to strongly quench a first portion of the probing spectrum and leave the second portion of the probing spectrum of the luminescent solid state material unquenched in order to have pronounced changes in the ratio of quenching depending on the concentration of the analyte. In practice, however, one has to accept quenching in all portions of the probing spectrum of the luminescent solid state material. According to a preferred embodiment of the invention, the first portion and the second portion of the probing spectrum of the luminescent solid state material are quenched in a ratio of quenching of at least 2:1, preferably at least 3:1, more preferably at least 5:1, even more preferably at least 10:1, and most preferably at least 20:1.

According to a preferred embodiment of the present invention, the probing spectrum of the luminescent solid state material has a first maximum and a second maximum and the indicator dye is selected to quench the first maximum of the probing spectrum and the second maximum of the probing spectrum in said ratio of quenching. When the probing spectrum has discernible maxima or peaks it is possible to select the indicator dye to quench only one of the maxima whereas the other maximum remains more or less unquenched so that high ratios of quenching can be achieved, which allows for precise determination of the concentration of the analyte.

In the context of this invention it is preferred when the luminescent solid state material is selected from the group consisting of Cr(III)-doped yttrium-aluminium borate (YAl₃(BO₃)₄), Cr(III)-doped gadolinium aluminium borate (GdAl₃(BO₃)₄), Cr(III)-doped yttrium aluminium garnet (Y₃Al₅O₁₂), calcium-copper silicate (Egyptian Blue = Ca-CᵤSi₄O₁₀) and strontium-copper silicate (SrCuSi₄O₁₀) or barium-copper silicate (Ba-CᵤSi₄O₁₀). These luminescent solid state materials stand out for their high chemical and photochemical stability and their relatively long fluorescent decay-times (>1 µs) favouring time-resolved measurements as described below in more detail. The time-resolved measurement allows for determining an analyte without potential influences from a fluorescent specimen or excitation light.

Preferred indicator dyes are selected from the group consisting of triphenylmethene dyes, xanthene dyes, diketopyrrolopyrrol dyes, azaborodipyrromethene and azadipyrromethene.

According to a preferred embodiment of the invention the luminescent solid state material is Cr(III)-doped gadolinium aluminium borate (GdAl₃(BO₃)₄) and the indicator dye is azaborodipyrromethene. This particular useful combination will be discussed below.

Another preferred embodiment of the invention is realised when the luminescent solid state material is calcium-copper silicate (Egyptian Blue = CaCᵤSi₄O₁₀)) and the indicator dye is m-Cresol purple. Also this combination of luminescent solid state material and indicator dye will be discussed below.

To realise a ready-to-use arrangement of an optical probe according to the invention, the optical probe is preferably devised such that the luminescent solid state material and the indicator dye are contained in layers on a solid carrier. According to a particularly simple and therefore preferred embodiment of the invention, the luminescent solid state material and the indicator dye are contained in one common layer on the solid carrier. For other applications the optical probe is advantageously devised such that the luminescent solid state material and the indicator dye are contained in separate layers on the solid carrier.

In order to increase the sensitivity of the optical probe according to the present invention the optical probe is preferably further developed such that the optical probe further has at least one additional layer containing a pigment, in particular TiO₂ particles. The TiO₂ particles in the additional layer reflect incoming light and therefore increase the optical path length in the optical probe thus increasing sensitivity.

The inventive method for quantitatively determining an analyte comprises the steps of
exposing an optical probe to an analyte, the optical probe containing a luminescent solid state material having an excitation spectrum and an emission spectrum, the optical probe further containing an indicator dye having different, analyte-dependent chemical forms and corresponding analyte-dependent absorption spectra wherein the absorption spectrum of at least one form of the indicator dye overlaps with the emission spectrum of the luminescent solid state material, the emission spectrum of the luminescent solid state material being broader than the overlapping absorption spectrum of the indicator dye,
exciting the optical probe with light at a single wavelength within the excitation spectrum of the luminescent solid state material
measuring emission of light at different wavelengths within the emission spectrum of the luminescent solid state material,
calculating a ratio of quenching of emission of light at said different wavelengths and comparing the ratio of quenching with a prerecorded calibration curve, wherein
the excitation and the measuring of the corresponding emission are carried out subsequently with a delay-time within the range of the luminescence decay-time of an excited state of the luminescent solid state material.

This represents a first embodiment of the inventive method.

The inventive concept of the present concept is, however, also realised by a method for quantitatively determining an analyte comprising the steps of
exposing an optical probe to an analyte, the optical probe containing a luminescent solid state material having an excitation spectrum and an emission spectrum and further containing an indicator dye having different, analyte-dependent chemical forms and corresponding analyte-dependent absorption spectra wherein the absorption spectrum of at least one form of the indicator dye overlaps with the excitation spectrum of the luminescent solid state material, the excitation spectrum of the luminescent solid state material being broader than the overlapping absorption spectrum of the indicator dye,
exciting the optical probe with light of different wavelengths within the excitation spectrum of the luminescent solid state material
measuring emission of light at a single wavelength within the emission spectrum of the luminescent solid state material,
calculating the ratio of quenching of the excitation by measuring emission at said single wavelength within the emission spectrum of the luminescent solid state material and
comparing the ratio of quenching with a prerecorded calibration curve, wherein
the excitation and the measuring of the corresponding emission are carried out subsequently with a delay-time within the range of the luminescence decay-time of an excited state of the luminescent solid state material.

This represents an alternative embodiment of the present invention

The inventive idea, thus, is to be seen in creating a probing spectrum of the luminescent solid state material by overlapping a relatively large excitation spectrum or emission spectrum of a luminescent solid state material with a comparatively narrower absorption spectrum of an indicator dye and either measuring the emission from the emission spectrum of the luminescent solid state material upon excitation or exciting the so created probing spectrum with light of different wavelengths and measuring the resulting emission. In the first embodiment, the excitation with light of a single wavelength will lead to emission of light at a greater wavelength, the emission being differently quenched at different wavelengths by the indicator dye depending on the concentration of the analyte. In the second embodiment the indicator dye will absorb part of the excitation light and accordingly reduce the emission of the luminescent solid state material at a greater wavelength. Since the indicator dye absorbs light in the excitation spectrum differently at different wavelengths, depending on the concentration of the analyte, the intensity of emission of light, i.e. luminescence will be different upon excitation at said different wavelengths. In the invention, it is vital that the excitation and the measuring of the corresponding emission are carried out subsequently with a delay-time within the range of the luminescence decay-time of an excited state of the luminescent solid state material. This is necessary for determining an analyte without potential influences from a fluorescent specimen.

The ratio of quenching that is calculated in both embodiments either from directly measured, quenched emission of light or from measuring emitted light where the energy transfer during excitation has been quenched allows for a precise quantitative determination of an analyte, i.e. its concentration in a medium, without having to take into account any external influences

To obtain particularly pronounced changes in the analyte-dependent ratio of quenching the invention is preferably carried out in such a way that the excitation or the measurement of emission at different wavelengths is carried out at maxima of the spectrum of the luminescent solid state material that is overlapped by the indicator dye. Pronounced changes in the ratios of quenching provide for a high sensitivity for measuring the concentration of the analyte to be determined.

A preferred way to carry out the invention is realised in that the wavelength of excitation is 590 nm and the wavelengths of measuring emission of light are 733 nm and 690 nm when the luminescent solid state material is Cr(III)-doped gadolinium aluminium borate and the indicator dye is azaborodipyrromethene. This method can further be improved when the excitation and the measuring of the emission of light are carried out with a delay-time between 1 □s and 4000 □s, preferably with a delay-time between 10 □s and 2000 □s, more preferably with a delay-time between 30 □s and 1000 □s and most preferably with a delay-time between 50 □s and 300 □s.

Another preferred embodiment of the present invention provides for the wavelengths of excitation being 667 nm and 596 nm and the wavelength of measuring emission of light being 910 nm when the luminescent solid state material is calcium-copper silicate (Egyptian Blue) and the indicator dye is m-Cresol purple. This method can further be improved when the excitation and the measuring of the emission of light preferably are carried out with a delay-time between 5□s and 40 □s, preferably between 10 □s and 30 □s, more preferably with a delay-time of 20 □s.

The invention will now be described in more detail by way of the following examples and in connection with the accompanying drawings in which
Fig.1a shows a cross section of an inventive optical probe for measuring pH,
Fig. 1b shows a cross section of an inventive optical probe for measuring CO ₂,
Fig. 1c shows a cross section of another inventive optical probe for measuring CO₂,
Fig. 2 shows spectral properties of the components of the pH sensor in THF solution,
Fig. 3a shows emission spectra of the pH sensor (□_{excitation} 590 nm) obtained in steady-state,
Fig. 3b shows emission spectra of the pH sensor (□_{excitation} 590 nm) obtained in time-resolved measurements (delay 20 µs)
Fig. 3c shows a pH-calibration curve for the time-resolved measurements
Fig. 4 shows spectral properties of the components of the inventive CO₂ sensor: 1 and 2 - absorption spectra of the pH indicator in the protonated and deprotonated forms, respectively; 3 and 4 - excitation and emission spectra of Egyptian Blue,
Fig.5a shows luminescence excitation spectra of the CO₂ sensing optical probe from example 2 (□ₑₘᵢₛₛᵢₒₙ 910 nm) obtained at 25°C,
Fig. 5b shows calibration plots for the luminescence intensity ratio R,
Fig. 6a show luminescence excitation spectra of the CO2 sensing optical probe from example 3 (□ₑₘᵢₛₛᵢₒₙ 910 nm) obtained at 25°C,
Fig. 6b show a calibration plot for the luminescence intensity ratio,
Fig. 7 show calibration plots for the CO₂-sensing optical probe from example 2 obtained upon excitation with a dually emitting orange/red LED at a modulation frequency of 916 Hz at 25°C,
Fig. 8 shows the general structure of indicator dyes that can be used in the present invention,

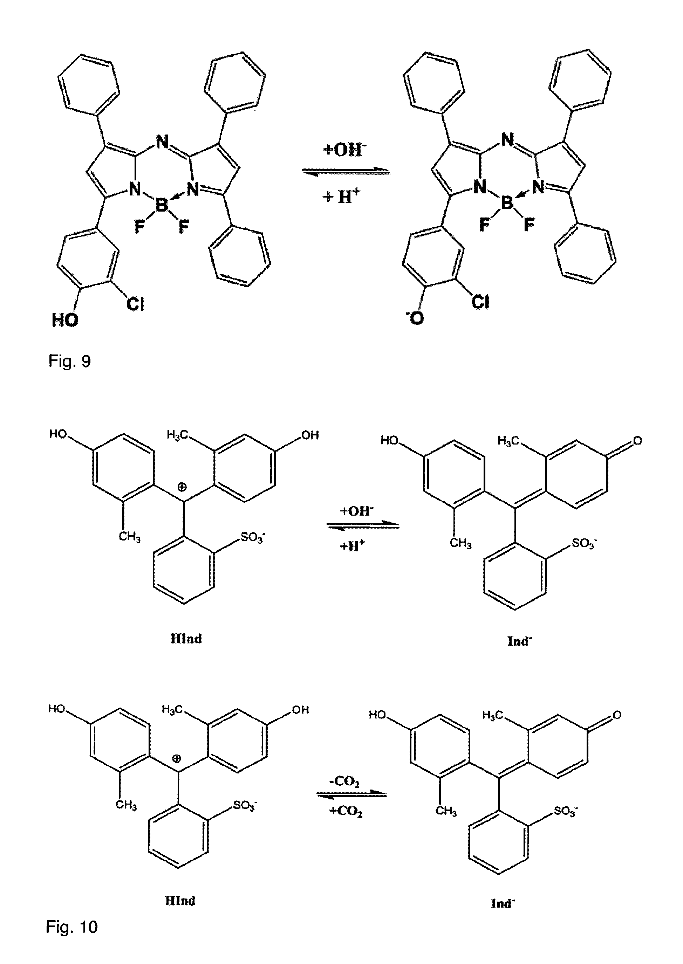

Fig. 9 shows the structural formula of aza-BODIPY, and
Fig. 10 shows the structural formulae of the triphenylmethene dyes in protonated and deprotonated forms.

### Example 1:

Optical probe for sensing pH based on 2-wavelength time-resolved measurement of the luminescence emission.

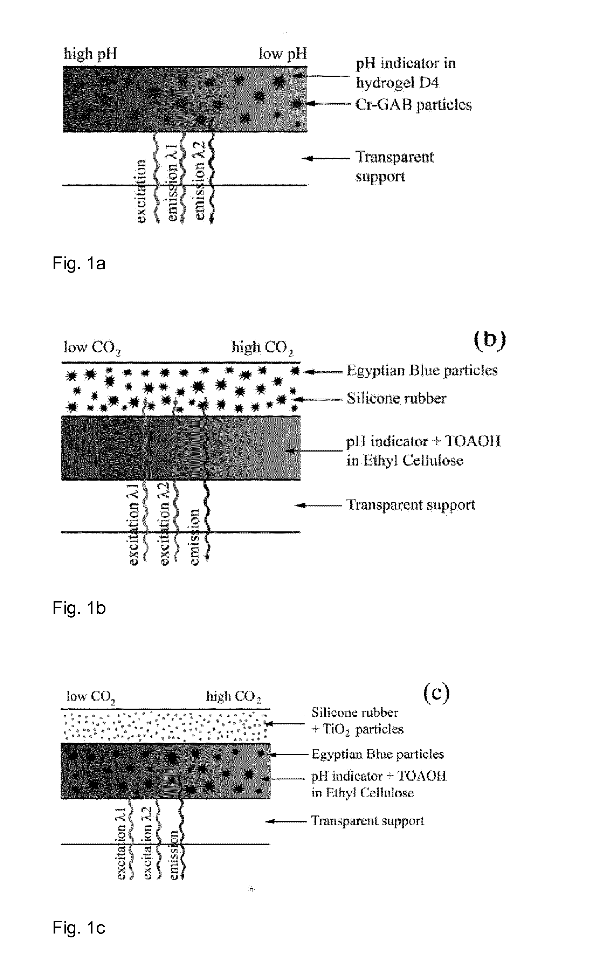

100 mg of hydrogel D4, 1 mg of the pH indicator dye BF₂ chelate of [5-(3-chloro-4-hydroxyphenyl)-3-phenyl-1 H-pyrrol-2-yl]-[5-phenyl-3-phenylpyrrol-2-ylidene]amine ("azaBODIPY"), and 100 mg of Cr(III)-doped gadolinium aluminium borate (Cr-GAB) microcrystals were dissolved/dispersed in 900 mg of EtOH:H₂O mixture (9:1 v/v). The mixture was knife-coated on a polyethylene terephthalate support to give about 8 µm-thick sensing layer after solvent evaporation. The sensor configuration is shown in Fig.1a.

### Measurements:

The emission spectra were measured on a Fluorolog 3 Horiba fluorescence spectrometer, with a wavelength of excitation at 590 nm corresponding to the maximum of the excitation spectrum of the Cr-GAB material. The emission was acquired in two modes: a steady-state mode and time-resolved measurement mode using a pulsed Xe-lamp (delay after pulse of 20 µs) to eliminate the background fluorescence. For the measurements, the optical probe was positioned in a flow-through cell and the buffers (ionic strength = IS 0.15M) were pumped through the cell. The temperature was kept constant at 25 °C for all the measurements.

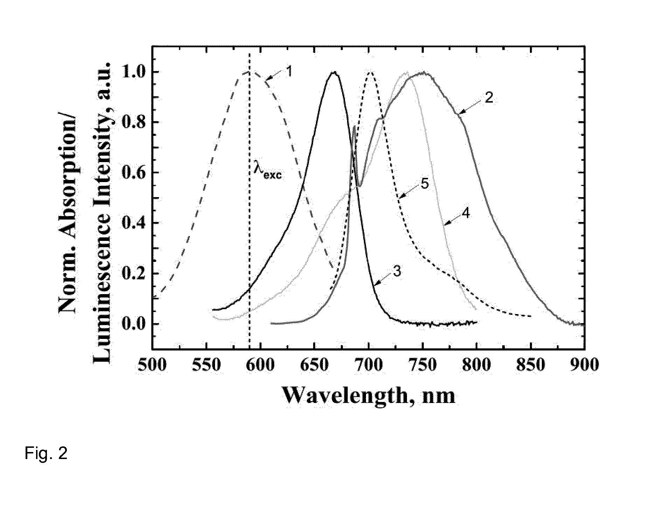

The absorption (excitation) and the emission spectra of the components are shown in Fig. 2 (1 and 2 - excitation and emission spectra of the Cr-GAB microcrystalline powder; 3 and 4 - absorption spectra of the pH indicator in the protonated and deprotonated forms, respectively; 5 - the emission spectrum of the protonated form of the pH indicator).

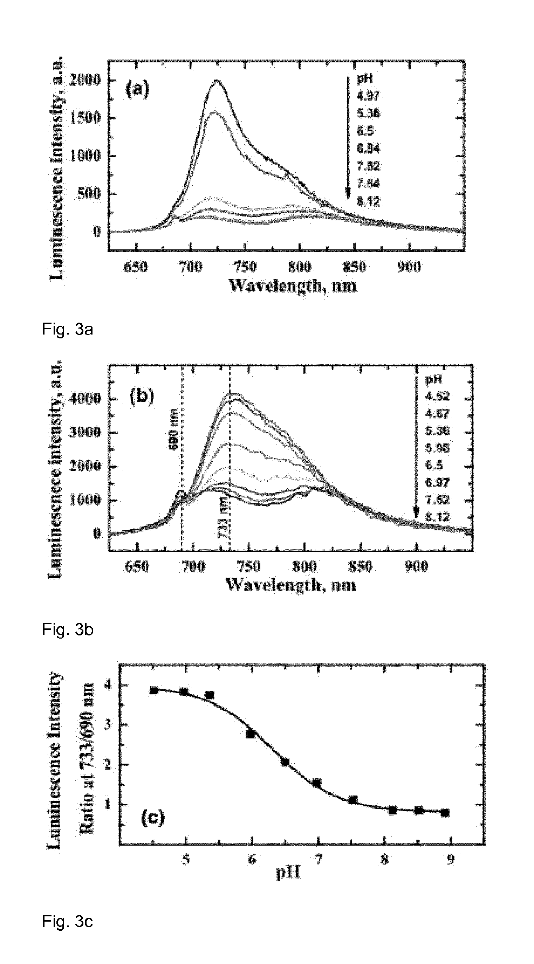

As can be seen in Fig. 2, the broad NIR emission from the Cr-GAB is observed upon excitation with orange light (590 nm). The emission spectrum of Cr-GAB overlaps with the absorption spectra of both the protonated and the deprotonated form of the pH indicator thus forming a probing spectrum. The protonated form of the pH indicator is also excitable at 590 nm and is highly fluorescent. The fluorescence spectrum overlaps with the emission spectrum of Cr-GAB which results in undesired background. This is revealed by Fig. 3a which shows the steady-state emission spectra of the sensing material when excited at 590 nm. Evidently, the emission of the optical probe is dominated by the fluorescence of the pH indicator which decreases upon deprotonation of the indicator dye due to quenching via photoinduced electron transfer.

Due to the long decay time of Cr-GAB (about 80 µs) the short-lived fluorescence can be completely eliminated in time-resolved measurements by applying a short delay after the excitation pulse. This delay was 20 µs in this example, but it can be much shorter with other excitation sources such as LEDs. Indeed, only the emission from the Cr-GAB phosphor is detected, and it is modulated by the absorption changes of the pH indicator (Fig. 3b). Ratiometric measurements become possible due to overlap of the absorption of the pH indicator with the emission spectrum of the luminescent solid state material resulting in analyte-dependent quenching of the emission spectrum. As can be seen in Fig. 3b, the maximum of the emission spectrum at 733 nm is strongly quenched by the absorption spectrum of the indicator dye, whereas the maximum at 690 nm remains more or less unchanged. There is, hence, a pronounced, analyte-dependent ratio of quenching that allows for sensitive determination of the analyte, when the ratio is compared to a calibration curve, which is obtained by plotting the ratio of the luminescence intensities obtained at 733 and 690 nm vs. pH. The calibration curve in this example is a typical sigmoidal curve (Fig. 3c)

### Example 2

Optical CO2-sensing probe based on 2-wavelength time-resolved measurement of the luminescence excitation.

The probe (Fig. 1b) was prepared according to the following procedure. 100 µL of tetraoctylammonium hydroxide (TOAOH) were added to 2 mg of m-Cresol Purple and the resulting solution was saturated with carbon dioxide. In another vial 0.2 g of ethyl cellulose (ethoxyl content 49%) were dissolved in a mixture of 1.52 g of ethanol and 2.28 g of toluene. The content of both vials was combined and the obtained mixture was knife-coated on a polyethylene terephthalate support and was allowed to dry for 1h at room temperature. The thickness of this CO₂-sensitive layer after solvent evaporation was estimated to be about 3.5 µm.

A mixture of silicone primers was obtained by mixing 800 mg of the vinyl-terminated polydimethylsiloxane (1000 cSt viscosity), 32 µl of methylhydrosiloxane-dimethylsiloxane copolymer, 2 µl of tetravinyltetramethyl cyclotetrasiloxane and 800 mg of hexane. 400 mg micro-powder of Egyptian Blue were dispersed in the mixture. Finally, 4 □l of the platinum complex catalyst were added and the composition was knife-coated onto the CO2-sensitive layer (75 µm spacer) and was allowed to cross-link at 60 °C for 20 min.

The CO₂-chemistry of the tetraoctylammonium cation, the indicator dye and the analyte CO₂ is represented by the following chemical equation.

TOA⁺Ind⁻ + CO₂+ H₂O ↔ Hlnd + TOA⁺HCO₃⁻

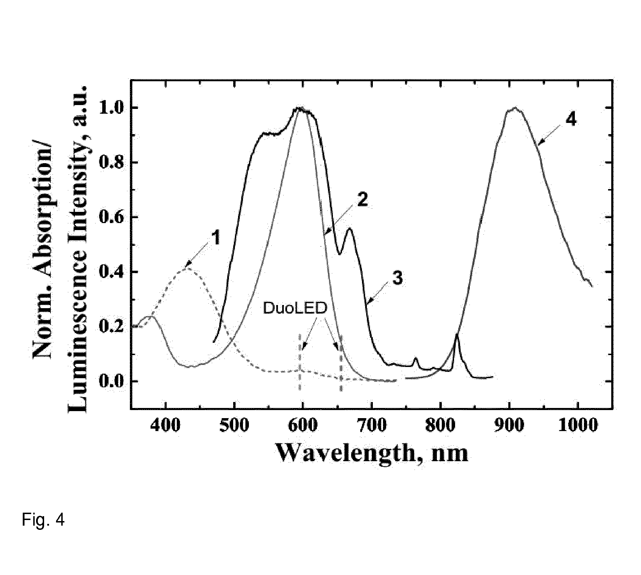

Spectral properties of the components are shown in Fig. 4.

Egyptian Blue is a near infra red emitting luminescent solid state material (□max = 910 nm) which has a broad excitation spectrum (500nm - 700 nm) in the visible part of the spectrum. The excitation spectrum partly overlaps with the absorption spectra of triphenylmethene dyes in the deprotonated form which is the basis of ratiometric interrogation of the optical probe via excitation. The pH indicator m-Cresol purple was dissolved in ethylcellulose along with a lipophilic base (tetraoctylammonium hydroxide). The long luminescence decay time (210 µs) allows elimination of background fluorescence in time-resolved measurements as already described. The excitation and the measuring of the corresponding emission were, hence, carried out subsequently with a delay-time within the range of the luminescence decay-time of an excited state of the luminescent solid state material. This allows for determining an analyte without potential influences from a fluorescent specimen.

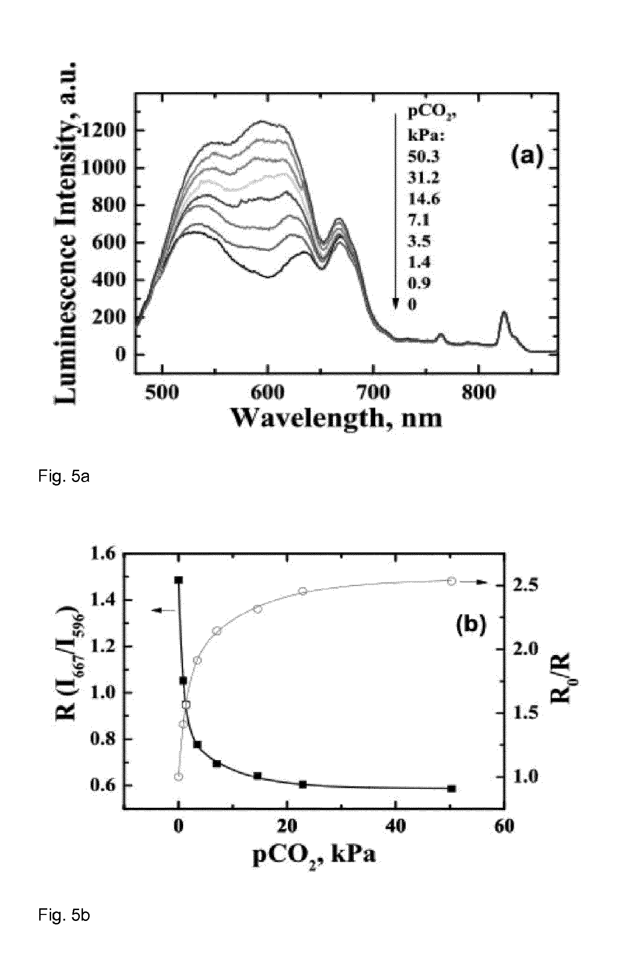

Figure 5a shows the excitation spectra of the CO₂-sensing material. As can be seen, a decrease in the signal with decreasing CO₂-partial pressure (pCO₂) is observed in the region of 525-625 nm, i.e. in the region corresponding to the absorption band of m-Cresol purple (deprotonated form). On the other hand, excitation from 650 to 700 nm is almost independent from pCO₂. The ratio of quenching of luminescence at 667 and 596 nm (R, Fig. 5b) is used as an analytical parameter.

The plot of the ratio at 0 kPa CO₂ and a pCO₂ is very similar to an intensity plot (I₀/I) of a typical carbon dioxide sensor (Fig. 5b)

### Example 3:

CO₂-sensing optical probe based on 2-wavelength time-resolved measurement of the luminescence excitation.

The optical probe (Fig. 1c) was prepared according to the following procedure. 100 µL of tetraoctylammonium hydroxide were added to 2 mg of m-Cresol Purple and the resulting solution was saturated with carbon dioxide. In the second vial 0.2 g of ethyl cellulose (ethoxyl content 49%) and 0.2 g of Si(CH₃)₃-modified Egyptian Blue were dissolved/dispersed in a mixture of 1.52 g of ethanol and 2.28 g of toluene. The contents of both vials were combined and the obtained mixture was knife-coated on a polyethylene terephthalate support and was allowed to dry for 1 h at room temperature to give about 5 µm-thick CO₂-sensitive layer after evaporation of the solvents.

The mixture of silicone primers was obtained by mixing 800 mg of the vinyl-terminated polydimethylsiloxane (1000 cSt viscosity), 32 µl of methylhydrosiloxane-dimethylsiloxane copolymer, 2 µl of tetravinyltetramethyl cyclotetrasiloxane and 800 mg of hexane. 0.25g of lipophilic titanium dioxide nanoparticles were dispersed in the mixture. Finally, 4 □l of the platinum complex catalyst were added and the composition was knife-coated onto the CO₂-sensitive layer (75 µm spacer) and was allowed to cross-link at 60 °C for 20 min.

The measurements were performed as described in Example 2.

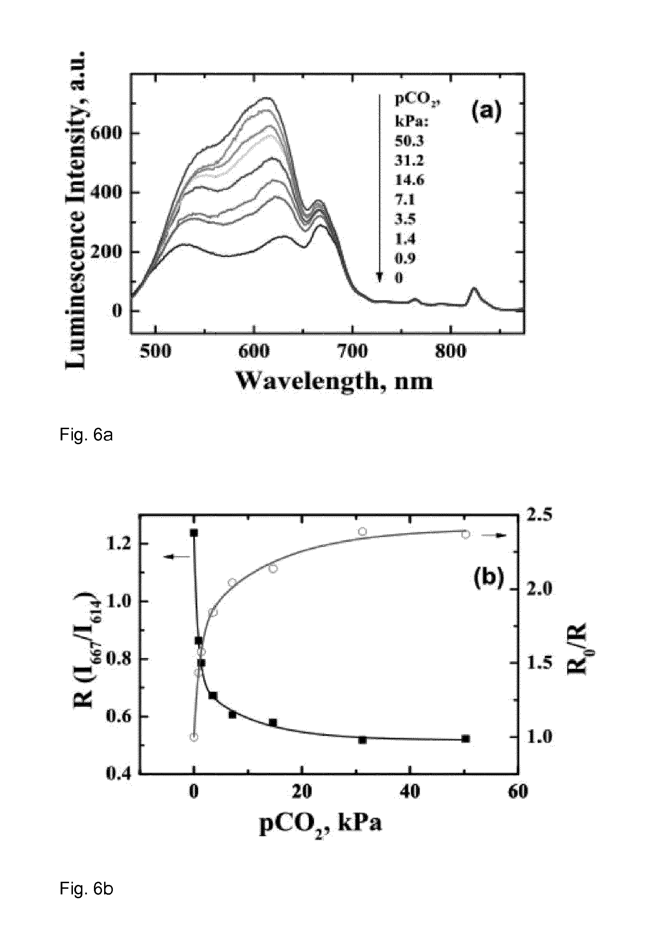

Fig. 6a shows the excitation spectra of the CO₂-sensing material and Fig. 6b the respective calibration plots. As can be seen, the spectral changes and the calibration plots are similar to those obtained in the example 2, but the relative changes in the luminescence intensity, i.e. the changes in the ratio of quenching, for the region 550-650 nm are more significant.

### Example 4

CO₂-sensing optical probe based on 2-wavelength time-resolved measurement of the luminescence excitation using a dually-emitting LED as an excitation source.

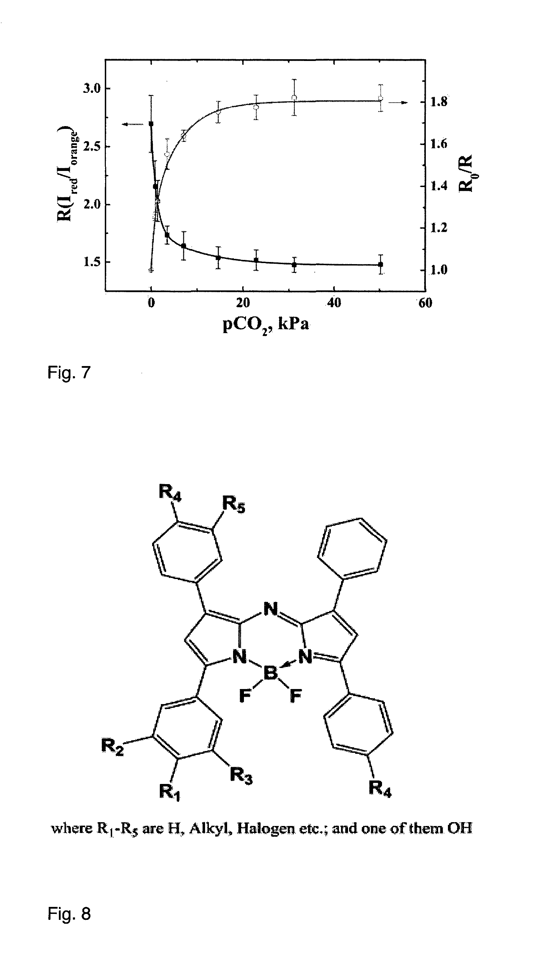

The sensor was prepared as described in the example 2. Excitation was performed with a dually-emitting LED: orange (□ₘₐₓ 594 nm) and red (□ₘₐₓ 653 nm). The LED light was sinusoidally modulated at a frequency of 916 Hz to eliminate the daylight component. The emission was detected with a photodiode after passing a long-pass RG-9 filter. Fig. 7 demonstrates feasibility of the approach using a dually-emitting LED as an excitation source. The calibration plots are similar to those obtained from the measurements of the excitation spectra (Fig. 5b). The approach demonstrates that realisation of compact sensor set-up for time-resolved ratiometric measurements is possible.

## Claims

1. Optical probe for quantitatively determining an analyte, the probe comprising
a luminescent solid state material as an internal light source having an excitation spectrum and an emission spectrum and
an indicator dye having different, analyte-dependent chemical forms and corresponding analyte-dependent absorption spectra,
wherein the absorption spectrum of at least one form of the indicator dye overlaps with either the excitation spectrum or the emission spectrum of the luminescent solid state material, referred to as a probing spectrum, and the luminescent solid state material has a probing spectrum broader than the absorption spectrum of the indicator dye,
wherein
by virtue of the indicator dye a first portion and a second portion of the probing spectrum of the luminescent solid state material are quenched to different extents, and wherein the decay time of the emission of the luminescent solid state material is longer than 1µs.

2. Optical probe according to claim 1, **characterised in that** the first portion and the second portion of the probing spectrum of the luminescent solid state material are quenched in a ratio of quenching of at least 2:1, preferably at least 3:1, more preferably at least 5:1, even more preferably at least 10:1, and most preferably at least 20:1.

3. Optical probe according to claims 1 or 2, **characterised in that** the probing spectrum of the luminescent solid state material has a first maximum and a second maximum and the indicator dye is selected to quench the first maximum of the probing spectrum and the second maximum of the probing spectrum in said ratio of quenching.

4. Optical probe according to one of claims 1, 2 or 3, **characterised in that** the luminescent solid state material is selected from the group consisting of Cr(III)-doped yttrium-aluminium borate (YAI₃(BO₃)₄), Cr(III)-doped gadolinium aluminium borate (GdAl₃(BO₃)₄), Cr(III)-doped yttrium aluminium garnet (Y₃Al₅O₁₂), calcium-copper silicate (Egyptian Blue = CaCuSi₄O₁₀) and strontium-copper silicate (SrCuSi₄O₁₀) or barium-copper silicate (BaCuSi₄O₁₀).

5. Optical probe according to one of claims 1 to 4, **characterised in that** the indicator dye is selected from the group consisting of triphenylmethane dyes, xanthene dyes, diketopyrrolopyrrol dyes, azaborodipyrromethene and azadipyrromethene.

6. Optical probe according to one of claims 1 to 3, **characterised in that** the luminescent solid state material is Cr(III)-doped gadolinium aluminium borate (GdAl₃(BO₃)₄) and the indicator dye is azaborodipyrromethene.

7. Optical probe according to one of claims 1 to 3, **characterised in that** the luminescent solid state material is calcium-copper silicate (Egyptian Blue = CaCuSi₄O₁₀) and the indicator dye is m-Cresol purple.

8. Optical probe according to any one of claims 1 to 7, **characterised in that** the luminescent solid state material and the indicator dye are contained in layers on a solid carrier.

9. Optical probe according to one of claims 1 to 8, **characterised in that** the luminescent solid state material and the indicator dye are contained in one common layer on the solid carrier.

10. Optical probe according to one of claims 1 to 8, **characterised in that** the luminescent solid state material and the indicator dye are contained in separate layers on the solid carrier.

11. Optical probe according to one of claims 1 to 10, **characterised in that** the optical probe further has at least one additional layer containing a pigment, in particular TiO₂ particles.

12. Method for quantitatively determining an analyte comprising the steps of
exposing an optical probe to an analyte, the optical probe containing a luminescent solid state material having an excitation spectrum and an emission spectrum, the optical probe further containing an indicator dye having different, analyte-dependent chemical forms and corresponding analyte-dependent absorption spectra wherein the absorption spectrum of at least one form of the indicator dye overlaps with the emission spectrum of the luminescent solid state material, the emission spectrum of the luminescent solid state material being broader than the overlapping absorption spectrum of the indicator dye,
exciting the optical probe with light at a single wavelength within the excitation spectrum of the luminescent solid state material
measuring emission of light at different wavelengths within the emission spectrum of the luminescent solid state material,
calculating a ratio of quenching of emission of light at said different wavelengths and
comparing the ratio of quenching with a prerecorded calibration curve, wherein
the excitation and the measuring of the corresponding emission are carried out subsequently with a delay-time within the range of the luminescence decay-time of an excited state of the luminescent solid state material.

13. Method for quantitatively determining an analyte comprising the steps of exposing an optical probe to an analyte, the optical probe containing a luminescent solid state material having an excitation spectrum and an emission spectrum and further containing an indicator dye having different, analyte-dependent chemical forms and corresponding analyte-dependent absorption spectra wherein the absorption spectrum of at least one form of the indicator dye overlaps with the excitation spectrum of the luminescent solid state material, the excitation spectrum of the luminescent solid state material being broader than the overlapping absorption spectrum of the indicator dye,
exciting the optical probe with light of different wavelengths within the excitation spectrum of the luminescent solid state material
measuring emission of light at a single wavelength within the emission spectrum of the luminescent solid state material,
calculating the ratio of quenching of the excitation by measuring emission at said single wavelength within the emission spectrum of the luminescent solid state material and
comparing the ratio of quenching with a prerecorded calibration curve, wherein
the excitation and the measuring of the corresponding emission are carried out subsequently with a delay-time within the range of the luminescence decay-time of an excited state of the luminescent solid state material.

14. Method according to claim 12 or 13, **characterised in that** the excitation or the measurement of emission at different wavelengths is carried out at maxima of the spectrum of the luminescent solid state material that is overlapped by the indicator dye.

15. Method according to one of claims 12 or 14, **characterised in that** the wavelength of excitation is 590 nm and the wavelengths of measuring emission of light are 733 nm and 690 nm when the luminescent solid state material is Cr(III)-doped gadolinium aluminium borate and the indicator dye is azaborodipyrromethene.

16. Method according to claim 15, **characterised in that** the excitation and the measuring of the emission of light are carried out with a delay-time between 1 □s and 4000 □s, preferably with a delay-time between 10 □s and 2000 □s, more preferably with a delay-time between 30 □s and 1000 □s and most preferably with a delay-time between 50 □s and 300 □s.

17. Method according to one of claims 13 to 14, **characterised in that** the wave-lenghts of excitation are 667 nm and 596 nm and the wavelength of measuring emission of light is 910 nm when the luminescent solid state material is calcium-copper silicate (Egyptian Blue) and the indicator dye is m-Cresol purple.

18. Method according to claim 17, **characterised in that** the excitation and the measuring of the emission of light are carried out with a delay-time between 5□s and 40 □s, preferably between 10 □s and 30 □s, more preferably with a delay-time of 20 □s.
